# EUROPÄISCHE PATENTSCHRIFT

(11) **EP 3 294 357 B1**
(45) Veröffentlichungstag und Bekanntmachung des Hinweises auf die Patenterteilung: **17.03.2021**
(21) Anmeldenummer: 16720853.7
(22) Anmeldetag: 06.05.2016
(51) Int. Cl.: A61L 27/42, A61L 24/00, A61F 2/28

(54) **KNOCHENERSATZMATERIALIEN, VERFAHREN ZUR HERSTELLUNG EINES KNOCHENERSATZMATERIALS SOWIE MEDIZINISCHE KITS ZUR BEHANDLUNG VON KNOCHENDEFEKTEN**
BONE REPLACEMENT MATERIALS, METHOD FOR PRODUCING A BONE REPLACEMENT MATERIAL AND MEDICAL KITS FOR THE TREATMENT OF BONE DEFECTS
MATÉRIAUX SUBSTITUTS OSSEUX, PROCÉDÉ DE PRODUCTION D'UN MATÉRIAU SUBSTITUT OSSEUX ET TROUSSES MÉDICALES DE TRAITEMENT DE MALFORMATIONS OSSEUSES

(30) Priorität: 15.05.2015 DE 102015209007
(43) Veröffentlichungstag der Anmeldung: 21.03.2018
(73) Patentinhaber: Aesculap AG, 78532 Tuttlingen (DE)
(72) Erfinder: GRUPP, Thomas, 78588 Denkingen (DE); HAGEN, Thomas, 78532 Tuttlingen (DE); ABELE, Wolfgang, 78532 Tuttlingen (DE)
(74) Vertreter: Patentanwälte Ruff, Wilhelm, Beier, Dauster & Partner mbB
(86) Internationale Anmeldenummer: PCT/EP2016/060218
(87) Internationale Veröffentlichungsnummer: WO 2016/184699

(56) Entgegenhaltungen:
- WO-A1-2013/153185
- US-A1- 2003 167 093

## Beschreibung

Die Erfindung betrifft Knochenersatzmaterialien.

Die Behandlung von Knochendefekten beruht üblicherweise auf der Verwendung von Implantaten und Knochenersatzmaterialien.

Bei der Behandlung von knöchernen Gelenkdefekten kommt - abhängig vom Grad des Defektes - neben dem eigentlichen Gelenkimplantat zusätzlich ein Implantat zur Defektauffüllung, d.h. zur Überbrückung des Knochendefekts, zum Einsatz. Letzteres wird dabei zwischen das Gelenkimplantat und dem noch gesunden Knochen positioniert. Alternativ kann auch ein Knochenersatzmaterial zwischen das Gelenkimplantat und dem intakten Knochen eingesetzt werden.

Implantate zur Überbrückung von Knochendefekten besitzen eine geometrisch genau definierte Form und setzten eine entsprechende Präparation des Knochendefektes zu deren knöchernen Aufnahme voraus. Dies ist mit großem Aufwand verbunden und - abhängig vom Defekttyp - nicht immer realisierbar.

Hinzu kommt, dass die mechanischen Eigenschaften der für eine Überbrückung von Knochendefekten vorgesehenen Implantate häufig nicht mit denen einer knöchernen Struktur vergleichbar sind. Dies gilt insbesondere für metallische Implantate. Die Übergänge von Metall auf Knochen unterliegen starken Belastungsschwankungen, wodurch ein langfristiges Verwachsen mit Knochengewebe und damit eine Stützfunktion des Implantats erschwert oder gar verhindert werden.

Keramische Implantate zur Behandlung von Knochendefekten sind beispielsweise aus den Druckschriften DE 28 27 529 C2, US 4,654,314, DE 35 31 144 C2, DE 600 00 877 T2, DE 600 33 025 T2, DE 100 18 394 B4 sowie DE 602 15 895 T2 bekannt. Diese Implantate bestehen in der Regel aus Calciumphosphatverbindungen. Calciumphosphat ist ein wesentlicher Bestandteil von Knochengewebe. Die mechanischen Eigenschaften von keramischen Implantaten sind daher eher mit denjenigen von natürlichem Knochengewebe vergleichbar als die mechanischen Eigenschaften von metallischen Implantaten.

Keramische Implantate sind jedoch - genauso wie metallische Implantate - mit dem Nachteil behaftet, dass sie sich in der Regel nicht an eine Defektform anpassen lassen. Entsprechendes gilt auch im Hinblick auf bereits eingesetzte Implantate. Eine Bearbeitung oder Anpassung der Implantate im Rahmen einer Revisionsoperation ist mithin nicht gegeben. Vielmehr ist es in diesen Fällen erforderlich, die Implantate vollständig unter Vergrößerung der Knochendefekte zu entfernen.

Aus der DE 10 2012 213 246 A1 sind Stützkörper in Form von Oligopoden zur Behandlung von Knochendefekten bekannt. Obgleich die beschriebenen Stützkörper eine leistungsstarke Alternative zur Behandlung von Knochendefekten darstellen, kann sich eine vollständige Auffüllung der Knochendefekte - abhängig von der Defektform - als schwierig gestalten.

Alternativ zu den bislang beschriebenen Implantaten werden sogenannte Knochenersatzmaterialien zur Behandlung, insbesondere Überbrückung, von Knochendefekten verwendet. Bei den Knochenersatzmaterialien kann es sich dabei um natürliche oder synthetische (artifizielle) Knochenersatzmaterialien handeln.

Beispiele für natürliche Knochenersatzmaterialien stellen Knochenspäne und Knochenchips dar. Nachteilig ist, dass sich diese Knochenersatzmaterialien ab einer gewissen Menge nicht mehr ausreichend in einem Defekt fixieren lassen. Es besteht das Risiko, dass sich einzelne Knochenspanteilchen bzw. -chips aus einer mit anderen Knochenspanteilchen bzw. -chips gebildeten Verbundstruktur ablösen, wodurch die Tragfähigkeit des Knochenersatzmaterials im Defektbereich geschmälert wird.

Die synthetischen bzw. artifiziellen Knochenersatzmaterialien enthalten häufig Knochenzementkomponenten, welche bei Kontakt mit Wasser zementartig abbinden. Entsprechende Knochenersatzmaterialien sind beispielsweise aus der EP 2 170 245 B1, DE 11 2010 001 628 T5 sowie DE 603 05 036 T2 bekannt.

Wie Knochenspäne oder -chips sind jedoch auch synthetische Knochenersatzmaterialien mit dem Nachteil behaftet, dass sie hinsichtlich ihrer Tragfähigkeit Limitierungen aufweisen. Dies gilt insbesondere im Falle von resorbierbaren Knochenersatzmaterialien. Insbesondere bei der Behandlung von Gelenkdefekten besteht daher ein Risiko, dass Kräfte, welche im Körper auf ein eingebautes Gelenkimplantat wirken, über die Knochenersatzmaterialien nur unzureichend in den verbliebenen gesunden Knochen eingeleitet werden. Die Knochenersatzmaterialien können die Kräfte nicht aufnehmen und versagen. Dies kann zum Ausfall des Implantatlagers und damit zu einer Lockerung des Implantats führen.

Aus der WO 2013/153185 A1 ist ein Implantat aus einem Faserverbundwerkstoff bekannt, welches neben einem resorbierbaren mineralischen Knochenzement verstärkende metallische Langfasern und/oder metallische Endlosfasern in Form mindestens einer gerüstgebenden, die Kontur des Implantats vorformenden Faserstruktur enthält.

Der vorliegenden Erfindung lag daher die Aufgabe zugrunde, Knochenersatzmaterialien bereitzustellen, welche die eingangs erwähnten Nachteile möglichst vermeiden.

Diese Aufgabe wird gelöst durch ein Knochenersatzmaterial mit den Merkmalen des unabhängigen Anspruchs 1. Bevorzugte Ausführungsformen sind in den abhängigen Ansprüchen definiert. Der Wortlaut sämtlicher Ansprüche wird hiermit durch ausdrückliche Bezugnahme zum Inhalt der vorliegenden Beschreibung gemacht. Weitere Erfindungsgegenstände sind in der Beschreibung offenbart.

Gemäß einem ersten Aspekt betrifft die Erfindung ein Knochenersatzmaterial bzw. einen Knochenersatzstoff.

Das Knochenersatzmaterial ist vorzugsweise zur Anwendung bei der Behandlung, insbesondere Überbrückung, von Knochendefekten vorgesehen. Mit anderen Worten handelt es sich bei dem Knochenersatzmaterial vorzugsweise um ein Knochenersatzmaterial zur Anwendung bei der Behandlung, insbesondere Überbrückung, von Knochendefekten.

Das Knochenersatzmaterial zeichnet sich besonders dadurch aus, dass es Stützkörper und eine modellierfähige sowie bei Kontakt mit Wasser oder einer wässrigen Flüssigkeit aushärtbare Masse aufweist.

Unter dem Ausdruck "modellierfähige Masse" soll im Sinne der vorliegenden Erfindung eine formbare, insbesondere plastisch formbare, oder duktile Masse verstanden werden, welche in einem nicht ausgehärteten Zustand eine passgenaue Auf- oder Befüllung eines Knochendefekts oder eines Teils eines Knochendefekts mit dem Knochenersatzmaterial bzw. ein passgenaues Modellieren des Knochenersatzmaterials an einen Knochendefekt oder einen Teil eines Knochendefekts ermöglicht.

Der Ausdruck "Stützkörper" definiert im Sinne der vorliegenden Erfindung Formkörper, welche zusammen mit der Masse maßgeblich für die Stütz- bzw. Tragfähigkeitseigenschaften des Knochenersatzmaterials verantwortlich sind.

Das Knochenersatzmaterial zeichnet sich insbesondere durch die folgenden Vorteile aus:
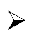 Das Knochenersatzmaterial besitzt eine gegenüber gattungsgemäßen Knochenersatzmaterialien verbesserte Tragfähigkeit.
   Die verbesserte Tragfähigkeit beruht unter anderem auf der zusätzlichen Verwendung von Stützkörpern, welche von der Masse bereits im nicht ausgehärteten Zustand zusammengehalten werden können.
   Eine zusätzliche Verbesserung der Tragfähigkeit ist durch Aushärtung der Masse erzielbar, wodurch die Stützkörper innerhalb der sich aushärtenden Masse verfestigt werden.
   Durch eine Druckbeaufschlagung des Knochenersatzmaterials, insbesondere der Masse, nach Auf- oder Befüllen eines Knochendefektes und insbesondere vor einem Aushärten der Masse kann der Zusammenhalt der Stützkörper zusätzlich erhöht werden.
   Darüber hinaus trägt die Masse als solche nach ihrer Aushärtung zu einer zusätzlichen Verbesserung der Tragfähigkeit des Knochenersatzmaterials bei.
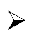 Das Knochenersatzmaterial bietet weiterhin die Möglichkeit einer Verfestigung in Belastungsrichtung und trägt damit insgesamt zu einer Erhöhung der Belastbarkeit des Knochenersatzmaterials nach Aushärtung der Masse bei.
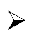 Die Masse erlaubt mit besonderem Vorteil ein einfaches Portionieren des Knochenersatzmaterials.
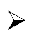 Des Weiteren erlaubt die Masse mit besonderem Vorteil ein passgenaues Auf- oder Befüllen von Knochendefekten mit dem Knochenersatzmaterial bzw. ein passgenaues Modellieren des Knochenersatzmaterials an Knochendefekte. Dadurch ist insbesondere auch eine Behandlung von geometrisch undefinierten Knochendefekten möglich.
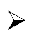 Durch Variation des Masseanteils in dem Knochenersatzmaterial kann der Grad des Auf- oder Befüllens eines Knochendefektes reguliert werden.
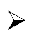 Weiterhin ist von Vorteil, dass auch im Zuge von Revisionen entstandene oder vergrößerte Knochendefekte besser aufgefüllt werden können. Dabei kann es ausreichend sein, die Knochendefekte ausschließlich mit dem Knochenersatzmaterial zu behandeln.
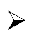 Alternativ kann das Knochenersatzmaterial zur Überbrückung eines Knochendefektes, insbesondere eines knöchernen Gelenkdefektes, vorgesehen sein.
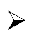 Ein weiterer Vorteil besteht darin, dass das Knochenersatzmaterial auch nach Aushärtung der Masse mechanisch, insbesondere spanend, bearbeitet werden kann und somit eine Entfernung des Knochenersatzmaterials bei Revisionsoperationen nicht erforderlich ist.

Vorzugsweise handelt es sich bei der Masse um eine bei Standardbedingungen, d.h. bei 25 °C und 101,325 kPa, modellierfähige Masse.

Bei der Masse handelt es sich gemäß einer bevorzugten Ausführungsform um eine pastöse Masse. Mit anderen Worten ist es erfindungsgemäß bevorzugt, wenn die Masse eine pastöse Konsistenz aufweist oder in Form einer Paste vorliegt.

Gemäß einer besonders bevorzugten Ausführungsform handelt es sich bei der Masse um eine knetbare Masse. Mit anderen Worten ist es erfindungsgemäß besonders bevorzugt, wenn die Masse eine knetbare Konsistenz aufweist oder in Form einer Knete bzw. Knetmasse vorliegt. Das Knochenersatzmaterial kann in dieser Ausführungsform auch als Knochenknete oder eine Art Knochenknete bezeichnet werden.

In einer besonders bevorzugten Ausführungsform sind die Stützkörper in der Masse enthalten.

In einer weiteren Ausführungsform sind die Stützkörper mit der Masse benetzt oder beschichtet.

Dabei können die Stützkörper nur teilweise, d.h. nur teilflächig, oder vollständig, d.h. vollflächig, mit der Masse benetzt oder beschichtet sein.

Insbesondere können die Stützkörper homogen verteilt in der Masse vorliegen.

Die Stützkörper sind ineinandergreifend, insbesondere miteinander verzahnbar, gegenseitig verblockbar oder gegenseitig verkeilbar, ausgebildet.

Die Stützkörper liegen in einem ineinandergreifenden Zustand vor. Erfindungsgemäß kann es insbesondere vorgesehen sein, dass die Stützkörper miteinander verzahnt, gegenseitig verblockt oder gegenseitig verkeilt vorliegen.

In einer weiteren Ausführungsform bilden die Stützkörper, bevorzugt durch gegenseitiges Ineinandergreifen, insbesondere gegenseitiges Verzahnen, Verblocken oder Verkeilen, eine dreidimensionale und insbesondere Hohlräume aufweisende Gerüststruktur aus. Die Hohlräume der Gerüststruktur sind dabei vorzugsweise mit der Masse befüllt. Diese Form der gegenseitigen Durchdringung von Masse und Stützkörper trägt zu einer weiteren Verbesserung der Stütz- bzw. Tragfähigkeitseigenschaften des Knochenersatzmaterials bei.

In einer weiteren unter Stütz- bzw. Tragfähigkeitsgesichtspunkten vorteilhaften Ausführungsform sind die Stützkörper porös ausgestaltet. Eine poröse Ausgestaltung der Stützkörper hat den Vorteil, dass sich die Stützkörper bei Belastung (leichter) komprimieren, insbesondere deformieren, lassen. Entsprechende zu einer Stützkörperkomprimierung führende Belastungen können beispielsweise bei einer Kraftbeaufschlagung der noch nicht ausgehärteten Masse durch einen behandelnden Mediziner oder während der Aushärtung der Masse auftreten. Dadurch lässt sich eine gegenseitige Verfestigung der Stützkörper zusätzlich verbessern, was wiederum verbesserte Tragfähigkeitseigenschaften des Knochenersatzmaterials zur Folge hat.

In einer weiteren Ausführungsform weisen die Stützkörper und/oder die Masse Fasern auf. Bei den Fasern kann es sich grundsätzlich um Kurz- und/oder Langfasern handeln. Bei den Fasern handelt es sich vorzugsweise um resorbierbare Fasern. Besonders bevorzugt handelt es sich bei den Fasern um Verstärkungsfasern, d.h. um Fasern, welche zur Verstärkung der Stützkörper und/oder der Masse vorgesehen sind. Mit anderen Worten ist es bevorzugt, wenn die Stützkörper und/oder die Masse faserverstärkt sind.

Grundsätzlich können die Stützkörper porös ausgestaltet sein, d.h. Poren besitzen. Bei den Poren kann es sich um geometrisch definierte oder undefinierte Poren handeln. Die Poren können einen Durchmesser, insbesondere einen mittleren Durchmesser, von 60 µm bis 500 µm, bevorzugt 100 µm bis 400 µm, aufweisen.

Die Stützkörper können weiterhin insbesondere geschlossen porig oder offenporig ausgestaltet sein. Eine offenporige Ausgestaltung der Stützkörper hat den zusätzlichen Vorteil, dass die Masse in die Poren der Stützkörper eindringen kann. Dadurch lassen sich die Stütz- bzw. Tragfähigkeitseigenschaften des Knochenersatzmaterials zusätzlich optimieren.

Erfindungsgemäß kann es daher bevorzugt sein, wenn ein Teil der Masse in Poren der Stützkörper enthalten ist.

Gemäß einer weiteren bevorzugten Ausführungsform weisen die Stützkörper Ausnehmungen oder Öffnungen auf. Die Ausnehmungen bzw. Öffnungen können grundsätzlich als Vertiefung ausgebildet sein. Erfindungsgemäß bevorzugt ist es jedoch, wenn die Ausnehmungen bzw. Öffnungen jeweils durchgehend ausgebildet sind, d.h. durchgehend offene Innenhohlräume bilden. Unter dem Ausdruck "durchgehend offene Innenhohlräume" sollen im Sinne der vorliegenden Erfindung Hohlräume in den Stützkörpern verstanden werden, welche nach Außen wenigstens zwei Öffnungen, insbesondere zwei gegenüberliegende Öffnungen, aufweisen.

Die Ausnehmungen bzw. Öffnungen können kreisförmig und/oder nicht kreisförmig, insbesondere polygonförmig, d.h. als Polygonzug, ausgebildet sein. Beispielsweise können die Ausnehmungen bzw. Öffnungen als Dreiecke, Vierecke, Fünfecke und/oder Sechsecke ausgebildet sein.

In einer weitergehenden Ausführungsform weisen die Ausnehmungen bzw. Öffnungen einen Durchmesser auf, welcher ein Hindurchführen wenigstens eines Zugelements gestattet. Die Ausnehmungen bzw. Öffnungen können beispielsweise einen Durchmesser von 0,01 mm bis 5 mm, insbesondere 0,1 mm bis 4 mm, bevorzugt 0,5 mm bis 3 mm, aufweisen. Durch ein Hindurchführen des wenigstens einen Zugelementes durch die Ausnehmungen bzw. Öffnungen der Stützkörper ist es möglich, die Stützkörper untereinander zu befestigen bzw. zu verzurren. Das wenigstens eine Zugelement kann die Stützkörper in einer eher zufälligen, d.h. randomisierten, oder aber in einer regelmäßigen Anordnung zusammenhalten.

Erfindungsgemäß kann es daher vorgesehen sein, dass das Knochenersatzmaterial ferner wenigstens ein Zugelement, d.h. ein Zugelement oder mehrere, insbesondere zwei oder mehr, Zugelemente aufweist. Das wenigstens eine Zugelement ist dabei vorzugsweise durch durchgehende Ausnehmungen bzw. Öffnungen der Stützkörper hindurchgeführt, wodurch die Stützkörper vorzugsweise miteinander verbunden, insbesondere verzurrt, werden.

Bei dem wenigstens einen Zugelement handelt es sich vorzugsweise um ein textiles Zugelement. Beispielsweise kann es sich bei dem wenigstens einen Zugelement um einen Faden (Zugfaden), insbesondere um einen monofilen oder multifilen Faden, handeln. Vorzugsweise handelt es sich bei dem wenigstens einen Zugelement um ein chirurgisches Nahtmaterial.

Alternativ kann es sich bei dem wenigstens einen Zugelement um ein textiles Flächengebilde, insbesondere um ein Gewirk, Geflecht, Gestrick, Gelege, Vlies oder Vliesstoff, handeln. Bevorzugt ist das wenigstens eine Zugelement ein Netz, insbesondere ein kleinporiges Netz, vorzugsweise ein Herniennetz. Durch ein Einbinden der Stützkörper in ein netzförmiges Zugelement kann eine regelmäßige Anordnung der Stützkörper angelegt werden. Ein netzförmig ausgebildetes Zugelement kann ähnlich einem Herniennetz feste Knotenpunkte besitzen und/oder einem lockeren Gelege entsprechen, welches in sich verschiebbar ist.

Das wenigstens eine Zugelement kann weiterhin ein resorbierbares und/oder nicht resorbierbares Material aufweisen oder aus einem resorbierbaren und/oder nicht resorbierbaren Material bestehen. Geeignete Materialien für das wenigstens eine Zugelement können ausgewählt sein aus der Gruppe aufweisend Metalle, Polymere, anorganische Materialien und Mischungen davon.

Weitere Vorteile, welche bei Verwendung wenigstens eines Zugelements realisiert werden können, werden nachfolgend beschrieben.

Die Verwendung wenigstens eines Zugelements ermöglicht ein Verzurren der Stützkörper, wodurch eine sofortige Festigkeitserhöhung der Stützkörper untereinander und damit des Knochenersatzmaterials erreicht werden kann. Dies kann mit besonderem Vorteil dazu führen, dass eine geringere Menge der erfindungsgemäß vorgesehenen Masse erforderlich ist, um ein stütz- bzw. tragfähiges Knochenersatzmaterial zu erhalten. Außerdem lässt sich durch eine derartige Festigkeitserhöhung der Stützkörper das Risiko senken, dass eine durch die Stützkörper gebildete Gerüststruktur nach einem Sprödbruch auseinanderbricht. Weiterhin kann durch ein Verzurren der Stützkörper mit besonderem Vorteil eine offenporigere Gerüststruktur realisiert werden. Weiterhin besteht die Möglichkeit, dass eine Zugelement-Stützkörper-Einheit (oder gegebenenfalls mehrere Zugelement-Stützkörper-Einheiten) an ein Implantat und/oder an einen Knochen fixiert und dadurch ortsstabil befestigt werden kann (können). Die Zugelement-Stützkörper-Einheit (oder Zugelement-Stützkörper-Einheiten) kann (können) durch die Befestigung an den Knochen und/oder das Implantat an den angefrischten Knochen angedrückt werden. Hierdurch ist eine optimale Anbindung an den Knochen möglich, und der resultierende Druck auf den Knochen fördert das Knochenwachstum. Die Kraftübertragung am Knochendefekt übernimmt bevorzugt das Implantat. Dadurch entfällt der Druckreiz, welcher den Knochen zum Knochenaufbau anregt (Stress Shielding). Dieser Druckreiz kann durch das bzw. die unter Druck zum Knochen stehenden Zugelement-Stützkörper-Einheiten aufgebaut werden.

In einer weiteren Ausführungsform sind die Stützkörper derart ausgestaltet, dass sie form-, kraft- und/oder stoffschlüssig miteinander verbindbar sind. Bevorzugt sind die Stützkörper derart ausgestaltet, dass sie formschlüssig miteinander verbindbar sind. Beispielsweise können die Stützköper derart ausgestaltet sein, dass sie über ein Stecksystem bzw. nach Art eines Stecksystems miteinander verbunden werden können. Das Stecksystem kann dabei auf einem sogenannten Zapfen-Loch-Prinzip beruhen, bevorzugt mit einem Hinterschnitt zur besseren Verankerung der Stützkörper. Hierzu können ein Teil der Stützkörper mit Zapfen und ein anderer Teil der Stützkörper mit passenden Zapflöchern oder Schlitzen versehen sein.

In einer weiteren Ausführungsform sind die Stützkörper form-, kraft- und/oder stoffschlüssig miteinander verbunden. Bevorzugt sind die Stützkörper formschlüssig miteinander verbunden. Beispielsweise können die Stützköper über ein Stecksystem bzw. nach Art eines Stecksystems miteinander verbunden sein. Bezüglich des Stecksystems wird auf den vorherigen Absatz Bezug genommen.

In einer weiteren Ausführungsform sind die Stützkörper derart ausgestaltet, dass sie form-, kraft- und/oder stoffschlüssig mit einem Implantat verbindbar sind. Bevorzugt sind die Stützkörper derart ausgestaltet, dass sie formschlüssig mit einem Implantat verbindbar sind. Beispielsweise können die Stützköper derart ausgestaltet sein, dass sie über ein Stecksystem bzw. nach Art eines Stecksystems mit einem Implantat verbunden werden können. Das Stecksystem kann dabei auf einem sogenannten Zapfen-Loch-Prinzip beruhen. Hierzu können die Stützkörper mit Zapfen versehen sein, und das Implantat kann komplementäre Zapfenlöcher oder Schlitze aufweisen. Die umgekehrten Verhältnisse können erfindungsgemäß ebenso möglich sein.

In einer weiteren Ausführungsform sind die Stützkörper über längliche Verbindungselemente miteinander verbunden. Bevorzugt ragen die Verbindungselemente hierzu in Ausnehmungen bzw. Öffnungen der Stützkörper. Bezüglich möglicher Ausgestaltungen der Ausnehmungen bzw. Öffnungen der Stützkörper wird auf die vorangegangenen Ausführungen Bezug genommen. Dabei können die Verbindungselemente und die Stützkörper ein gleiches Material aufweisen oder aus einem gleichen Material bestehen. Bevorzugt ist es jedoch, wenn die Verbindungselemente und die Stützkörper verschiedene Materialien aufweisen oder aus verschiedenen Materialien bestehen. Vorzugsweise weisen die Stützkörper ein spröderes Material auf oder bestehen aus einem spröderen Material als die Verbindungselemente. Geeignete Materialien für die Verbindungselemente stellen resorbierbare, insbesondere langzeitresorbierbare, Polymere, wie beispielsweise Polydioxanon, Polyhydroxyalkanoate wie Poly-(3-Hydroxybutyrat) und/oder Poly-(4-Hydroxybutyrat), Polyesterurethane und Mischungen davon dar.

Die Stützkörper weisen in einer weiteren Ausführungsform eine Partikelgröße von 2 mm bis 20 mm, bevorzugt 3 mm bis 10 mm, auf.

In einer weiteren Ausführungsform liegen die Stützkörper in unterschiedlichen Größen, insbesondere Partikelgrößen, vor. Dadurch kann eine gegenseitige Verblockung, Verzahnung oder Verkeilung der Stützköper erhöht werden.

Grundsätzlich können die Stützkörper eine definierte oder undefinierte Form besitzen.

Erfindungsgemäß bevorzugt ist es, wenn die Stützkörper eine definierte Form besitzen. Eine definierte Form der Stützkörper kann eine gegenseitige Verankerung bzw. Verfestigung der Stützkörper erleichtern.

Beispielsweise können die Stützkörper eine drei- bis zehneckige, insbesondere eine vier-, fünf- oder sechseckige, Außenkontur aufweisen.

Weiterhin können die Stützkörper in Umfangsrichtung eine sphärisch, insbesondere kugelförmig oder ellipsoidförmig, verlaufende Außenkontur aufweisen.

Weiterhin können die Stützkörper (im Wesentlichen) zylinderförmig, insbesondere kreiszylinderförmig, ausgestaltet sein.

Insbesondere können die Stützkörper (im Wesentlichen) hohlzylinderförmig, insbesondere hohlkreiszylinderförmig, ausgestaltet sein.

Weiterhin können die Stützkörper eine (im Wesentlichen) würfelförmige Außenkontur aufweisen.

Weiterhin können die Stützkörper eine polyederförmig, insbesondere tetra- und/oder oktaederförmig, verlaufende Außenkontur aufweisen.

Gemäß einer besonders bevorzugten Ausführungsform handelt es sich bei den Stützkörpern oder einem Teil der Stützkörper um Oligopoden.

Die Oligopoden können konusförmig und insbesondere rotationssymmetrisch ausgebildete Beine aufweisen. Die Beine können dabei einen Konuswinkel von 5° bis 25°, insbesondere 7° bis 15°, aufweisen.

Weiterhin können die Oligopoden Beine mit einer Länge von 0.5 mm bis 5 mm, insbesondere 1.5 mm bis 2.5 mm, aufweisen.

Des Weiteren können die Oligopoden Beine mit einem mittleren Durchmesser von 0.2 mm bis 3 mm, insbesondere 0.3 mm bis 0.7 mm, aufweisen.

Die Oligopoden können ferner ausgewählt sein aus der Gruppe umfassend Tripoden, Tetrapoden, Pentapoden, Hexapoden, Heptapoden, Oktapoden und Mischungen davon.

Erfindungsgemäß bevorzugt ist es, wenn es sich bei den Stützkörpern oder einem Teil der Stützkörper um Tetrapoden handelt. Eine tetrapodenförmige Ausgestaltung erlaubt ein besonders wirkungsvolles gegenseitiges Ineinandergreifen der Stützkörper.

Grundsätzlich kann das Knochenersatzmaterial resorbierbar, teilresorbierbar oder nicht resorbierbar sein.

Bevorzugt ist das Knochenersatzmaterial wenigstens teilresorbierbar, d.h. teilresorbierbar oder vollständig resorbierbar.

Ist das Knochenersatzmaterial teilresorbierbar, so ist es bevorzugt, wenn die Stützkörper nicht resorbierbar sind. Bezüglich insoweit geeigneter Materialien wird auf die im Folgenden erläuterten Materialien Bezug genommen.

Im Falle eines vollständig resorbierbaren Knochenersatzmaterials kann es weiterhin von Vorteil sein, wenn sich die für die Masse sowie für die Stützkörper vorgesehenen Materialien hinsichtlich ihres Resorptionsverhaltens, insbesondere hinsichtlich ihrer Resorptionsdauer, voneinander unterscheiden.

Die Stützkörper können ein resorbierbares, teilresorbierbares oder nicht resorbierbares Material aufweisen oder aus einem solchen Material bestehen.

Um die Tragfähigkeit des Knochenersatzmaterials zusätzlich zu verbessern, ist es in einer weiteren Ausführungsform vorgesehen, dass die Stützkörper ein nicht resorbierbares Material aufweisen oder aus einem solchen Material bestehen. Ein derartiges Material ist vorzugsweise ausgewählt aus der Gruppe umfassend oder bestehend aus Metalle, Legierungen, Boride, Carbide, Nitride, Silicide, Polymere und Mischungen davon.

Geeignete Metalle können ausgewählt sein aus der Gruppe umfassend oder bestehend aus Titan, Tantal, Niob, Wolfram und Zirconium.

Geeignete Legierungen können ausgewählt sein aus der Gruppe umfassend oder bestehend aus Titan-Legierungen, Tantal-Legierungen, Niob-Legierungen, Wolfram-Legierungen und Zirconium-Legierungen.

Geeignete Boride können ausgewählt sein aus der Gruppe umfassend oder bestehend aus Niobborid, Wolframborid und Mischungen davon.

Geeignete Carbide können ausgewählt sein aus der Gruppe umfassend oder bestehend aus Aluminiumcarbid, Borcarbid, Niobcarbid, Siliciumcarbid, Tantalcarbid, Titancarbid, Wolframcarbid, Vanadiumcarbid, Zirconiumcarbid und Mischungen davon.

Geeignete Nitride können ausgewählt sein aus der Gruppe umfassend oder bestehend aus Bornitrid, Chromnitrid, Siliciumnitrid, Tantalnitrid, Titannitrid, Zirconiumnitrid und Mischungen davon.

Geeignete Silicide können ausgewählt sein aus der Gruppe umfassend oder bestehend aus Tantalsilicid, Wolframsilicid, Zirconiumsilicid und Mischungen davon.

Geeignete (nicht resorbierbare) Polymere können ausgewählt sein aus der Gruppe umfassend oder bestehend aus Polymethylmethacrylat (PMMA), Polyetheretherketon (PEEK), Polyvinylchlorid (PVC), Polyethylen (PE), Polyethylen hoher Dichte (HDPE), ultrahochmolekulares Polyethylen (UHMWPE), Polystyrol, Polyester wie beispielsweise Polyethylenterephthalat, Polyamide, Polypropylen (PP), fluorierte, insbesondere perfluorierte, Polymere (wie Polytetrafluorethylen (PTFE), Polytrifluorchlorethylen (PTFCE), Polyvinylfluorid (PVF), Polyvinylidendifluorid (PVDF), Hexafluorpropylen und/oder Tetrafluorethylen), Copolymere davon und Blends davon.

In einer weiteren Ausführungsform weisen die Stützkörper ein resorbierbares Material auf oder bestehen aus einem resorbierbaren Material, welches ausgewählt ist aus der Gruppe umfassend oder bestehend aus Calciumverbindungen wie Calciumphosphate, Magnesiumverbindungen wie Magnesiumphosphate, Polymere und Mischungen davon.

Geeignete (resorbierbare) Polymere können ausgewählt sein aus der Gruppe umfassend oder bestehend aus Polyhydroxyalkanoate, Polylactid, Polyglycolid, Poly-ε-Caprolacton, Polytrimethylencarbonat, Poly-p-dioxanon, Proteine, extrazelluläre Proteine, Collagen, Elastin, Retikulin, Fibronektin, Gelatine, Polysaccharide, Mucopolysaccharide, Hyaluronsäure, Heparin, Heparansulfat, Chondroitinsulfat, Dermatansulfat, Keratansulfat, Copolymere davon und Mischungen, insbesondere Blends, davon.

Bezüglich geeigneter Calcium- und/oder Magnesiumverbindungen wird auf die im Folgenden im Zusammenhang der Masse erwähnten Verbindungen Bezug genommen.

Wie bereits erwähnt, ist die erfindungsgemäß vorgesehene Masse bei Kontakt mit Wasser oder einer wässrigen Flüssigkeit aushärtbar. Bei der wässrigen Flüssigkeit handelt es sich vorzugsweise um Körperflüssigkeit, insbesondere Knochengewebsflüssigkeit. Alternativ oder zusätzlich kann es sich bei der wässrigen Flüssigkeit um eine wässrige Lösung, insbesondere wässrige wirkstoffhaltige Lösung, wie beispielsweise wässrige Salzlösung oder wässrige arzneistoffhaltige Lösung, oder eine wässrige Suspension handeln.

Erfindungsgemäß kann es grundsätzlich vorgesehen sein, die Aushärtung der Masse vor einem Einbringen, während eines Einbringens oder nach einem Einbringen des Knochenersatzmaterials in einen Knochendefekt durch Zugabe von Wasser oder einer wässrigen Flüssigkeit aktiv zu initiieren.

Bevorzugt ist es jedoch, wenn nach einem Einbringen des Knochenersatzmaterials in einen Knochendefekt eine Aushärtung der Masse durch Kontakt mit Knochengewebsflüssigkeit, insbesondere mit interstitieller Knochengewebsflüssigkeit, erfolgt. Dies ist insoweit vorteilhaft, als einem behandelnden Mediziner in der Regel mehr Zeit für ein passgenaues Auffüllen eines Knochendefektes zur Verfügung steht, ohne dass ein vorzeitiges Aushärten der Masse und damit des Knochenersatzmaterials zu befürchten ist.

Grundsätzlich kann die Masse bei Kontakt mit Wasser oder einer wässrigen Flüssigkeit innerhalb von 15 min bis 72 h, bevorzugt 45 min bis 18 h, aushärtbar sein.

Die Masse kann weiterhin resorbierbar, teilresorbierbar oder nicht resorbierbar sein.

Gemäß einer besonders bevorzugten Ausführungsform weist die Masse einen bei Kontakt mit Wasser oder einer wässrigen Flüssigkeit aushärtbaren Feststoff auf. Die Aushärtung des Feststoffes beruht vorzugsweise auf einer zementartigen Abbindereaktion mit Wasser oder einer wässrigen Flüssigkeit. Insbesondere kann es sich bei dem Feststoff um eine Knochenzementvorstufe handeln. Bezüglich der wässrigen Flüssigkeit wird auf die vorangegangenen Ausführungen Bezug genommen.

Bei dem Feststoff handelt es sich gemäß einer weiteren Ausführungsform um einen mineralischen Feststoff.

Der Feststoff weist vorzugsweise eine Calciumverbindung, insbesondere eine Calciumphosphatverbindung, und/oder eine Magnesiumverbindung, insbesondere eine Magnesiumphosphatverbindung, auf.

Die Calciumverbindung ist in einer weiteren Ausführungsform ausgewählt aus der Gruppe umfassend oder bestehend aus Monocalciumphosphat-Monohydrat (MCPM), Monocalciumphosphat-Anhydrid (MCPA), Dicalciumphosphat-Anhydrid (DCPA), Dicalciumphosphat-Dihydrat (DCPD), Octacalciumphosphat (OCP), α-Tricalciumphosphat (a-TCP), β-Tricalciumphosphat (β-TCP), amorphes Calciumphosphat (ACP), Hydroxylapatit (HA), Calcium-defizientes Hydroxylapatit (CdHA), substituiertes Hydroxylapatit, nicht stöchiometrisches Hydroxylapatit, nanoskaliges Hydroxylapatit, Tetracalciumphosphat (TTCP), Calciumsulfat (CaSO₄), Calciumsulfat-Hemihydrat (CaSO₄ x 0.5 H₂O), Calciumsulfat-Dihydrat (CaSO₄ x 2 H₂O), Calciumoxid (CaO), Calciumhydroxid (Ca(OH)₂), Calciumcarbonat (CaCO₃), Calciumglycerophosphat, Calciumcitrat, Calciumlactat, Calciumacetat, Calciumtartrat, Calciumchlorid (CaCl₂), Calciumsilicate und Mischungen davon.

Die Magnesiumverbindung ist in einer weiteren Ausführungsform ausgewählt aus der Gruppe umfassend oder bestehend aus Magnesiumhydrogenphosphat (MgHPO₄) in Form der Hydrate oder als wasserfreie Substanz, Trimagnesiumphosphat (Mg₃(PO₄)₂), Magnesiumdihydrogenphosphat (Mg(H₂PO₄)₂) in Form der Hydrate oder als wasserfreie Substanz, Magnesiumchlorid (MgCl₂) in Form der Hydrate oder als wasserfreie Substanz, Magnesiumglycerophosphat, Magnesiumhydroxid (Mg(OH)₂), Magnesiumhydroxidcarbonat (beispielsweise als 4 MgCO₃ x Mg(OH)₂ x 5 H₂O), Magnesiumoxid (MgO), Magnesiumcitrate (Mg₃(C₆H₅O₇)₂) oder Mg(C₆H₆O₇)), Calcium-Magnesiumcarbonat (CaMg(CO₃)₂, Dolomit und Mischungen davon.

Bei dem Feststoff handelt es sich vorzugsweise um einen partikulären Feststoff, insbesondere um ein Pulver.

In einer weiteren Ausführungsform weist der Feststoff einen Anteil von 20 Gew.-% bis 90 Gew.-%, vorzugsweise 65 Gew.-% bis 85 Gew.-%, auf, bezogen auf das Gesamtgewicht der Masse.

Ein besonders hoher Feststoffanteil der Masse kann durch eine breite Partikelgrößenverteilung erzielt werden, insbesondere wenn > 10 % des Feststoffes aus Partikeln < 10 µm und wenn > 10 % des Feststoffes aus Partikeln > 50 µm bestehen.

Erfindungsgemäß kann es weiterhin bevorzugt sein, dass die Stützkörper sowie die Masse ein gleiches resorbierbares Material aufweisen. Bei dem resorbierbaren Material handelt es sich vorzugsweise um eine Calciumverbindung, insbesondere Calciumphosphatverbindung, und/oder um eine Magnesiumverbindung, insbesondere Magnesiumphosphatverbindung. Insoweit wird vollständig auf die vorangegangenen Ausführungen Bezug genommen.

Die Masse weist in einer weiteren Ausführungsform, insbesondere neben einem Feststoff, wie in den vorangegangenen Ausführungsformen beschrieben, eine organische Trägerflüssigkeit auf. Die Trägerflüssigkeit ist vorzugsweise dazu vorgesehen, der Masse eine modellierfähige Konsistenz zu verleihen und/oder die Aushärtung der Masse bei Kontakt mit Wasser oder einer wässrigen Flüssigkeit zu verlangsamen. Insbesondere ist die Trägerflüssigkeit als Träger für einen Feststoff, wie in den vorangegangenen Ausführungsformen beschrieben, vorgesehen.

Bei der organischen Trägerflüssigkeit handelt es sich vorzugsweise um eine in Wasser nicht lösliche, d.h. wasserunlösliche, organische Trägerflüssigkeit.

In einer alternativen Ausführungsform handelt es sich bei der organischen Trägerflüssigkeit um eine organische Trägerflüssigkeit, welche nur gering in Wasser löslich ist. Bevorzugt ist die Trägerflüssigkeit bei dieser Ausführungsform < 25%, insbesondere < 10%, bevorzugt < 5%, bezogen auf das Volumen, in Wasser löslich.

Bei der organischen Trägerflüssigkeit kann es sich weiterhin insbesondere um ein Öl handeln.

Vorzugsweise ist die organische Trägerflüssigkeit ausgewählt aus der Gruppe umfassend oder bestehend aus Glycerintriacetat, Glycerintributyrat, Glycerintrioleat, Glycerindioleat, Glycerinmonooleat, Caprylcaprat, Decyloleat, Isopropylmyristat, Isopropylpalmitat, Ölsäure, Oleylalkohol, Oleyloleat, kurzkettige Triglyceride, mittelkettige Triglyceride (beispielsweise Myritol® 318 PH, Miglyol® 810, Miglyol® 812, Miglyol® 829), kurz- und mittelkettige Fettsäureester des Propylenglycols (beispielsweise Miglyol® 840), Ethylbenzoylacetat, Ethylbutyrat, Ethylbutyrylacetat, Ethyloleat, Ethylcaproat, Ethylcaprylat, Ethylcaprat, Ethyllaurat, Ethyllävulinat, Ethylmyristat, Ethylpalmitat, Ethyllinoleat, Ethylstearat, Ricinolsäure, Linolsäure, Linolesäure, Arachinsäure, Oleinsäure, Ethylarachidat, α-Tocopherol, β-Tocopherol, γ-Tocopherol, δ-Tocopherol, Benzylalkohol, Benzylbenzoat, Diethylbutylmalonat, Diethylenglycoldibutylether, Diethylmalonat, Diethylphenylmalonat, Diethylphthalat, Diethylsebacat, Diethylsubarat, Diethylsuccinat, Dibutylmaleinat, Dibutylphthalat, Lecithin, Paraffinöl, Petrolatum, flüssige Paraffine, Ester der Sebacinsäure, insbesondere Sebacinsäuredibutylester, Sebacinsäurediethylester, Sebacinsäurediisopropylester, Sebacinsäuredioctylester und Mischungen davon.

In einer weiteren Ausführungsform weist die Masse ferner einen Aushärtungsregulator, insbesondere einen Aushärtungsbeschleuniger oder -verzögerer, auf.

Bei dem Aushärtungsbeschleuniger kann es sich beispielsweise um ein Tensid handeln.

Das Tensid kann gemäß einer weitergehenden Ausführungsform ausgewählt sein aus der Gruppe umfassend oder bestehend aus Fettsäuren sowie deren Salze, Ester von Fettsäuren sowie deren Salze, Carbonsäureether, Alkylsulfate, Alkylethersulfate, Alkylsulfonate, Sulfosuccinate, Mono-Alkylphosphorsäureester, Di-Alkylphosphorsäureester, Acylaminosäuren sowie deren Salze, Alkylaminsalze, Alkylimidazoline, Tetraalkylammoniumsalze, Tetraarylammoniumsalze, heterocyclische Ammoniumsalze, ethoxylierte Amine, amphotere Tenside, Lecithine, Fettalkohole, ethoxylierte Fettalkohole, Ethylenoxid-Blockcopolymere, Propylenoxid-Blockcopolymere, Alkylphenolethoxylate, Alkylpolyglucoside, ethoxylierte Fette und Öle, Alkanolamide, ethoxylierte Alkanolamide, Polyethylenglycolfettsäureester, Glycolester, Sorbitanester (Mono- und Triester), Zuckerester, Ester-Tenside, Ether-Tenside und Mischungen davon.

Beispielsweise kann das Tensid ausgewählt sein aus der Gruppe umfassend oder bestehend aus Natriumlaurylsulfat, Glycerinmonooleat, Polysorbat 20, 21, 40, 60, 61, 65, 80, 81, 85, 120, Sorbitandiisostearat, Sorbitandioleat, Sorbitanmonoisostearat, Sorbitanmonolaurat, Sorbitanmonooleat, Sorbitanmonopalmitat, Sorbitanmonostearat, Sorbitantrioleat, Sorbitantrilaurat, Sorbitantricaprylat, Sorbitantricaprat, Isopropylmyristat, Lecithin, Lysolecithine, Oleinsäure, Olsäure, Polyethylenglycolmonocetylether, Polyethylenglycolmonostearylether, Polyethylenglycolmonolaurylether, Polyethylenglycolmonooleylether, polyethoxyliertes Rizinusöl, Polyoxyl-40-Stearat, Polyoxyl-50-Stearat, Ascorbylpalmitat, Cetylphosphat und Mischungen davon.

In einer weiteren Ausführungsform weist die Masse ferner einen Aushärtungsverzögerer auf, welcher ausgewählt ist aus der Gruppe umfassend oder bestehend aus Pyrophosphate, Citrate, Magnesiumionen, Calciumcarbonat und Mischungen davon.

In einer weiteren Ausführungsform weist die Masse einen Anteil von 20 Gew.-% bis 80 Gew.-%, bevorzugt 30 Gew.-% bis 70 Gew.-%, auf, bezogen auf das Gesamtgewicht des Knochenersatzmaterials.

In einer weiteren Ausführungsform weisen die Stützkörper einen Anteil von 20 Gew.-% bis 80 Gew.-%, bevorzugt 30 Gew.-% bis 70 Gew.-%, auf, bezogen auf das Gesamtgewicht des Knochenersatzmaterials.

Gemäß einer weiteren Ausführungsform weist das Knochenersatzmaterial, insbesondere die Masse und/oder die Stützkörper, einen Wirkstoff auf. Der Wirkstoff kann ausgewählt sein aus der Gruppe umfassend oder bestehend aus physiologisch aktive Substanzen, Antibiotika, entzündungshemmende Arzneimittel, Zytostatika, knochenmorphogenetische Proteine (bone morphogenetic proteins, BMPs), Arzneimittel gegen Osteoporose und Mischungen davon.

Beispielsweise kann der Wirkstoff ausgewählt sein aus der Gruppe umfassend oder bestehend aus BMP 1, BMP 2, BMP 3, BMP 3B, BMP 4, BMP 5, BMP 6, BMP 7, BMP 8A, BMP 8B, BMP 10, BMP 15, Interferone, Interleukine, insbesondere Interleukin-1β, Interleukin-6, koloniestimulierende Faktoren, Chemokine, Genamycin, Polyhexamethylenbiguanid (PHMB), Silberverbindungen, insbesondere Silbersalze, bevorzugt in Form von Nanopartikeln, und Mischungen davon.

Das Knochenersatzmaterial ist gemäß einer weiteren Ausführungsform zur Anwendung
a. bei der Behandlung, insbesondere Überbrückung, von unfallbedingten Knochendefekten,
b. bei der Behandlung, insbesondere Überbrückung, von entzündlichen Knochendefekten,
c. bei der Behandlung, insbesondere Überbrückung, von tumorbedingten Knochendefekten,
d. bei der Behandlung, insbesondere Überbrückung, von knöchernen Gelenkdefekten, insbesondere Hüftgelenk- und/oder Kniegelenkdefekten, bevorzugt von Becken-, Hüftpfannen-, Hüftkopf-, Oberschenkelhals-, Oberschenkel-, Wadenbein- und/oder Schienbeindefekten,
e. bei der Auffüllung von Knochendefekten, bevorzugt in einem gelenknahen Bereich,
f. beim Ersatz und/oder bei der Abstützung bzw. Unterfütterung von Abstützimplantaten, wie beispielsweise Spacerimplantaten, Stützschalen oder Unterlegplatten (Wedges), und/oder
g. bei der Rekonstruktion von Knochengewebe vorgesehen.

Mit anderen Worten handelt es sich bei dem Knochenersatzmaterial gemäß einer weiteren Ausführungsform um ein Knochenersatzmaterial zur Anwendung
a. bei der Behandlung, insbesondere Überbrückung, von unfallbedingten Knochendefekten,
b. bei der Behandlung, insbesondere Überbrückung, von entzündlichen Knochendefekten,
c. bei der Behandlung, insbesondere Überbrückung, von tumorbedingten Knochendefekten,
d. bei der Behandlung, insbesondere Überbrückung, von knöchernen Gelenkdefekten, insbesondere Hüftgelenk- und/oder Kniegelenkdefekten, bevorzugt von Becken-, Hüftpfannen-, Hüftkopf-, Oberschenkelhals-, Oberschenkel-, Wadenbein- und/oder Schienbeindefekten,
e. bei der Auffüllung von Knochendefekten, bevorzugt in einem gelenknahen Bereich,
f. beim Ersatz und/oder bei der Abstützung bzw. Unterfütterung von Abstützimplantaten, wie beispielsweise Spacerimplantaten, Stützschalen oder Unterlegplatten (Wedges), und/oder
g. bei der Rekonstruktion von Knochengewebe.

Weitere Merkmale und Vorteile ergeben sich aus den nachfolgend beschriebenen bevorzugten Ausführungsformen in Form eines Beispiels sowie den Ansprüchen, ohne die Erfindung hierauf zu beschränken.

### Ausführungsbeispiel

Mittels eines Gießverfahrens wurden aus einem kurz- und langfaserverstärken Calciumphosphat-Zement (CaP-Zement, PL Powder Liquid Typ) Tetrapoden gegossen. Hierzu wurden zweiteilige Silikonformen mittels Metallprototypen hergestellt. In diese Formen wurden die Tetrapoden gegossen. Die Tetrapoden hatten eine Ausdehnung von 8 mm. Nach Aushärtung der Calcumphosphat-Tetrapoden wurden diese mit einem pastenförmigen Calciumphospat-Zement (CaP-Zement, Velox, InnoTERE) eingegossen. Die so hergestellten Proben wiesen eine 20 % höhere Biegefestigkeit auf als die Proben, welche nur mit einem pastenförmige Calciumphosphat-Zement hergestellt wurden.

## Patentansprüche

1. Knochenersatzmaterial, aufweisend Stützkörper und eine modellierfähige sowie bei Kontakt mit Wasser oder einer wässrigen Flüssigkeit aushärtbare Masse, **dadurch gekennzeichnet, dass** die Stützkörper ineinandergreifend ausgebildet sind.

2. Knochenersatzmaterial nach Anspruch 1, **dadurch gekennzeichnet, dass** es sich bei der Masse um eine pastöse oder knetbare Masse handelt.

3. Knochenersatzmaterial nach Anspruch 1 oder 2, **dadurch gekennzeichnet, dass** die Stützkörper in der Masse enthalten sind.

4. Knochenersatzmaterial nach einem der vorhergehenden Ansprüche, **dadurch gekennzeichnet, dass** die Stützkörper durch gegenseitiges Ineinandergreifen eine dreidimensionale, Hohlräume aufweisende Gerüststruktur ausbilden.

5. Knochenersatzmaterial nach Anspruch4, **dadurch gekennzeichnet, dass** die Hohlräume mit der Masse befüllt sind.

6. Knochenersatzmaterial nach einem der vorhergehenden Ansprüche, **dadurch gekennzeichnet, dass** ein Teil der Masse in Poren der Stützkörper enthalten ist.

7. Knochenersatzmaterial nach einem der vorhergehenden Ansprüche, **dadurch gekennzeichnet, dass** die Stützkörper eine Partikelgröße von 2 mm bis 20 mm, bevorzugt 3 mm bis 10 mm, aufweisen.

8. Knochenersatzmaterial nach einem der vorhergehenden Ansprüche, **dadurch gekennzeichnet, dass** es sich bei den Stützkörpern um Oligopoden, vorzugsweise Tetrapoden, handelt.

9. Knochenersatzmaterial nach einem der vorhergehenden Ansprüche, **dadurch gekennzeichnet, dass** das Knochenersatzmaterial wenigstens teilresorbierbar ist.

10. Knochenersatzmaterial nach einem der vorhergehenden Ansprüche, **dadurch gekennzeichnet, dass** die Stützkörper ein Material aufweisen oder aus einem Material bestehen, welches ausgewählt ist aus der Gruppe umfassend Calciumverbindungen wie Calciumphosphate, Magnesiumverbindungen wie Magnesiumphosphate, Metalle, Legierungen, Boride, Carbide, Nitride, Silicide, Polymere und Mischungen davon.

11. Knochenersatzmaterial nach einem der vorhergehenden Ansprüche, **dadurch gekennzeichnet, dass** die Masse einen bei Kontakt mit Wasser oder einer wässrigen Flüssigkeit aushärtbaren Feststoff aufweist.

12. Knochenersatzmaterial nach Anspruch11, **dadurch gekennzeichnet, dass** der Feststoff eine Calciumverbindung, insbesondere ausgewählt aus der Gruppe umfassend Monocalciumphosphat-Monohydrat (MCPM), Monocalciumphosphat-Anhydrid (MCPA), Dicalciumphosphat-Anhydrid (DCPA), Dicalciumphosphat-Dihydrat (DCPD), Octacalciumphosphat (OCP), α-Tricalciumphosphat (α-TCP), β-Tricalciumphosphat (β-TCP), amorphes Calciumphosphat (ACP), Hydroxylapatit (HA), Calcium-defizientes Hydroxylapatit (CdHA), substituiertes Hydroxylapatit, nicht stöchiometrisches Hydroxylapatit, nanoskaliges Hydroxylapatit, Tetracalcium¬phosphat (TTCP), Calciumsulfat (CaSO4), Calciumsulfat-Hemihydrat (CaSO4 x 0.5 H2O), Calciumsulfat-Dihydrat (CaSO4 x 2 H2O), Calciumoxid (CaO), Calciumhydroxid (Ca(OH)2), Calciumcarbonat (CaCO3), Calciumglycerophosphat, Calciumcitrat, Calciumlactat, Calciumacetat, Calciumtartrat, Calciumchlorid (CaCl2), Calciumsilicate und Mischungen davon, und/oder eine Magnesiumverbindung, insbesondere ausgewählt aus der Gruppe umfassend Magnesiumhydrogenphosphat (MgHPO4) in Form der Hydrate oder als wasserfreie Substanz, Trimagnesiumphosphat (Mg3(PO4)2), Magnesiumdihydrogenphosphat (Mg(H2PO4)2) in Form der Hydrate oder als wasserfreie Substanz, Magnesiumchlorid (MgCl2) in Form der Hydrate oder als wasserfreie Substanz, Magnesiumglycerophosphat, Magnesiumhydroxid (Mg(OH)2), Magnesiumhydroxidcarbonat (beispielsweise als 4 MgCO3 x Mg(OH)2 x 5 H2O), Magnesiumoxid (MgO), Magnesiumcitrate (Mg3(C6H5O7)2) oder Mg(C6H6O7)), Calcium-Magnesiumcarbonat (CaMg(CO3)2, Dolomit und Mischungen davon, aufweist.

13. Knochenersatzmaterial nach Anspruch 11 oder 12, **dadurch gekennzeichnet, dass** der Feststoff einen Anteil von 20 Gew.-% bis 80 Gew.-%, vorzugsweise 30 Gew.-% bis 70 Gew.-%, aufweist, bezogen auf das Gesamtgewicht der Masse.

14. Knochenersatzmaterial nach einem der vorhergehenden Ansprüche, **dadurch gekennzeichnet, dass** die Stützkörper und die Masse ein gleiches resorbierbares Material aufweisen, wobei es sich bei dem resorbierbaren Material vorzugsweise um eine Calcium- und/oder Magnesiumverbindung, insbesondere Calciumphosphat- und/oder Magnesiumphosphatverbindung, handelt.

15. Knochenersatzmaterial nach einem der vorhergehenden Ansprüche, **dadurch gekennzeichnet, dass** die Masse ferner eine in Wasser nicht lösliche, organische Trägerflüssigkeit, insbesondere ausgewählt aus der Gruppe umfassend Glycerintriacetat, Glycerintributyrat, Glycerintrioleat, Glycerindioleat, Glycerinmonooleat, Caprylcaprat, Decyloleat, Isopropy-Imyristat, Isopropylpalmitat, Ölsäure, Oleylalkohol, Oleyloleat, kurzkettige Triglyceride, mittelkettige Triglyceride, kurz- und mittelkettige Fettsäureester des Propylenglycols, Ethylbenzoylacetat, Ethylbutyrat, Ethylbutyrylacetat, Ethyloleat, Ethylcaproat, Ethylcaprylat, Ethylcaprat, Ethyllaurat, Ethyllävulinat, Ethylmyristat, Ethylpalmitat, Ethyllinoleat, Ethylstearat, Ricinolsäure, Linolsäure, Linolesäure, Arachinsäure, Oleinsäure, Ethylarachidat, α-Tocopherol, β-Tocopherol, γ-Tocopherol, δ-Tocopherol, Benzylalkohol, Benzylbenzoat, Diethylbutylmalonat, Diethylenglycoldibutylether, Diethylmalonat, Diethylphenylmalonat, Diethylphthalat, Diethylsebacat, Diethylsubarat, Diethylsuccinat, Dibutylmaleinat, Dibutylphthalat, Lecithin, Paraffinöl, Petrolatum, flüssige Paraffine, Ester der Sebacinsäure, insbesondere Sebacinsäuredibutylester, Sebacinsäurediethylester, Sebacinsäurediisopropylester, Sebacinsäuredioctylester und Mischungen davon, aufweist.

## Claims

1. Bone replacement material having reinforcing elements and a modelable mass which is curable on contact with water or an aqueous liquid, **characterized in that** the reinforcing elements are in intermeshing form.

2. Bone replacement material according to claim 1, **characterized in that** the mass is a pasty or kneadable mass.

3. Bone replacement material according to claim 1 or 2, **characterized in that** the reinforcing elements are present in the mass.

4. Bone replacement material according to any of the preceding claims, **characterized in that** the reinforcing elements form a three-dimensional framework structure having cavities by intermeshing with one another.

5. Bone replacement material according to claim 4, **characterized in that** the cavities are filled by the mass.

6. Bone replacement material according to any of the preceding claims, **characterized in that** a part of the mass is present in pores of the reinforcing elements.

7. Bone replacement material according to any of the preceding claims, **characterized in that** the reinforcing elements have a particle size of 2 mm to 20 mm, preferably 3 mm to 10 mm.

8. Bone replacement material according to any of the preceding claims, **characterized in that** the reinforcing elements are oligopods, preferably tetrapods.

9. Bone replacement material according to any of the preceding claims, **characterized in that** the bone replacement material is at least partly absorbable.

10. Bone replacement material according to any of the preceding claims, **characterized in that** the reinforcing elements comprise or consist of a material selected from the group comprising calcium compounds such as calcium phosphates, magnesium compounds such as magnesium phosphates, metals, alloys, borides, carbides, nitrides, silicides, polymers and mixtures thereof.

11. Bone replacement material according to any of the preceding claims, **characterized in that** the mass includes a solid material which is curable on contact with water or an aqueous liquid.

12. Bone replacement material according to claim 11, **characterized in that** the solid material includes a calcium compound, in particular selected from the group comprising monocalcium phosphate monohydrate (MCPM), monocalcium phosphate anhydride (MCPA), dicalcium phosphate anhydride (DCPA), dicalcium phosphate dihydrate (DCPD), octacalcium phosphate (OCP), α-thcalcium phosphate (α-TCP), β-tricalcium phosphate (β-TCP), amorphous calcium phosphate (ACP), hydroxylapatite (HA), calcium-deficient hydroxylapatite (CdHA), substituted hydroxylapatite, non-stoichiometric hydroxylapatite, nanoscale hydroxylapatite, tetracalcium phosphate (TTCP), calcium sulfate (CaSO₄), calcium sulfate hemihydrate (CaSO₄ x 0.5 H₂O), calcium sulfate dihydrate (CaSO₄ x 2 H₂O), calcium oxide (CaO), calcium hydroxide (Ca(OH)₂), calcium carbonate (CaCO₃), calcium glycerophosphate, calcium citrate, calcium lactate, calcium acetate, calcium tartrate, calcium chloride (CaCl₂), calcium silicates and mixtures thereof, and/or a magnesium compound, in particular selected from the group comprising magnesium hydrogenphosphate (MgHPO₄) in the form of the hydrates or as an anhydrous substance, trimagnesium phosphate (Mg₃(PO₄)₂), magnesium dihydrogenphosphate (Mg(H₂PO₄)₂) in the form of the hydrates or as an anhydrous substance, magnesium chloride (MgCl₂) in the form of the hydrates or as an anhydrous substance, magnesium glycerophosphate, magnesium hydroxide (Mg(OH)₂), magnesium hydroxide carbonate (for example in the form of 4 MgCO₃ x Mg(OH)₂ x 5 H₂O), magnesium oxide (MgO), magnesium citrates (Mg₃(C₆H₅O₇)₂) or Mg(C₆H₆O₇)), calcium magnesium carbonate (CaMg(CO₃)₂), dolomite and mixtures thereof.

13. Bone replacement material according to claim 11 or 12, **characterized in that** the solid material has a proportion of 20% by weight to 80% by weight, preferably 30% by weight to 70% by weight, based on the total weight of the mass.

14. Bone replacement material according to any of the preceding claims, **characterized in that** the reinforcing elements and the mass include the same absorbable material, the absorbable material preferably being a calcium compound and/or magnesium compound, in particular calcium phosphate compound and/or magnesium phosphate compound.

15. Bone replacement material according to any of the preceding claims, **characterized in that** the mass further includes a water-insoluble organic carrier liquid, in particular selected from the group comprising glycerol triacetate, glycerol tributyrate, glycerol trioleate, glycerol dioleate, glycerol monooleate, capryl caprate, decyl oleate, isopropyl myristate, isopropyl palmitate, oleic acid, oleyl alcohol, oleyl oleate, short-chain triglycerides, mid-chain triglycerides, short- and mid-chain fatty acid esters of propylene glycol, ethyl benzoylacetate, ethyl butyrate, ethyl butyrylacetate, ethyl oleate, ethyl caproate, ethyl caprylate, ethyl caprate, ethyl laurate, ethyl levulinate, ethyl myristate, ethyl palmitate, ethyl linoleate, ethyl stearate, ricinoleic acid, linoleic acid, linolenic acid, arachic acid, oleic acid, ethyl arachidate, α-tocopherol, β-tocopherol, γ-tocopherol, δ-tocopherol, benzyl alcohol, benzyl benzoate, diethyl butylmalonate, diethylene glycol dibutyl ether, diethyl malonate, diethyl phenylmalonate, diethyl phthalate, diethyl sebacate, diethyl subarate, diethyl succinate, dibutyl maleate, dibutyl phthalate, lecithin, paraffin oil, petrolatum, liquid paraffins, esters of sebacic acid, especially dibutyl sebacate, diethyl sebacate, diisopropyl sebacate, dioctyl sebacate and mixtures thereof.

## Revendications

1. Matériau de remplacement d'os, présentant des corps de soutien et une masse modelable ainsi que durcissable au contact de l'eau ou d'un liquide aqueux, **caractérisé en ce que** les corps de soutien sont réalisés de façon à s'emboîter.

2. Matériau de remplacement d'os selon la revendication 1, **caractérisé en ce que** la masse est une masse pâteuse ou malléable.

3. Matériau de remplacement d'os selon la revendication 1 ou 2, **caractérisé en ce que** les corps de soutien sont contenus dans la masse.

4. Matériau de remplacement d'os selon l'une quelconque des revendications précédentes, **caractérisé en ce que** les corps de soutien sont réalisés par emboîtement mutuel d'une structure d'armature tridimensionnelle présentant des espaces creux.

5. Matériau de replacement d'os selon la revendication 4, **caractérisé en ce que** les espaces creux sont remplis avec la masse.

6. Matériau de remplacement d'os selon l'une quelconque des revendications précédentes, **caractérisé en ce qu'**une partie de la masse est contenue dans les pores des corps de soutien.

7. Matériau de remplacement d'os selon l'une quelconque des revendications précédentes, **caractérisé en ce que** les corps de soutien présentent une grosseur de particule de 2 mm à 20 mm, préférablement 3 mm à 10 mm.

8. Matériau de remplacement d'os selon l'une quelconque des revendications précédentes, **caractérisé en ce que** les corps de soutien sont des oligopodes, de préférence des tétrapodes.

9. Matériau de remplacement d'os selon l'une quelconque des revendications précédentes, **caractérisé en ce que** le matériau de remplacement d'os est au moins partiellement résorbable.

10. Matériau de remplacement d'os selon l'une quelconque des revendications précédentes, **caractérisé en ce que** les corps de soutien présentent un matériau ou sont constitués d'un matériau, qui est choisi dans le groupe constitué par des composés du calcium comme les phosphates de calcium, des composés du magnésium comme les phosphates de magnésium, des métaux, des alliages, des borures, des carbures, des nitrures, des siliciures, des polymères et des mélanges correspondants.

11. Matériau de remplacement d'os selon l'une quelconque des revendications précédentes, **caractérisé en ce que** la masse présente un solide durcissable par contact avec de l'eau ou un liquide aqueux.

12. Matériau de remplacement d'os selon la revendication 11, **caractérisé en ce que** le solide présente un composé du calcium, en particulier choisi dans le groupe comprenant le monohydrate de phosphate monocalcique (MCPM), l'anhydride de phosphate monocalcique (MCPA), l'anhydride de phosphate dicalcique (DCPA), le dihydrate de phosphate dicalcique (DCPD), le phosphate octacalcique (OCP), le phosphate α-tricalcique (α-TCP), le phosphate β-tricalcique (β-TCP), le phosphate de calcium amorphe (ACP), l'hydroxyapatite (HA), l'hydroxyapatite déficiente en calcium (CdHa), l'hydroxyapatite substituée, hydroxyapatite non stoechiométrique, l'hydroxyapatite nanométrique, le phosphate tétracalcique (TTCP), le sulfate de calcium (CaSO₄), l'hémihydrate de sulfate de calcium (CaSO₄ x 0,5 H₂O), le dihydrate de sulfate de calcium (CaSO₄ x 2 H₂O), l'oxyde de calcium (CaO), l'hydroxyde de calcium (Ca(OH)₂), le carbonate de calcium (CaCO₃), le glycérophosphate de calcium, le citrate de calcium, le lactate de calcium, l'acétate de calcium, le tartrate de calcium, le chlorure de calcium (CaCl₂), des silicates de calcium et des mélanges correspondants, et/ou un composé du magnésium, en particulier choisi dans le groupe comprenant l'hydrogénophosphate de magnésium (MgHPO₄) sous forme d'hydrates ou en tant que substance anhydre, le phosphate de trimagnésium (Mg₃(PO₄)₂), le dihydrogénophosphate de magnésium (Mg(H₂PO₄)₂) sous forme d'hydrates ou en tant que substance anhydre, le chlorure de magnésium (MgCl₂) sous forme d'hydrates ou en tant que substance anhydre, le glycérophosphate de magnésium, l'hydroxyde de magnésium (Mg(OH)₂), l'hydroxyde carbonate de magnésium (par exemple en tant que 4 MgCO₃ x Mg(OH)₂ x 5 H₂O), l'oxyde de magnésium (MgO), le citrate de magnésium (Mg₃(C₆H₅O₇)₂) ou Mg(C₆H₆O₇)), le carbonate de calcium-magnésium (CaMg(CO₃)₂), la dolomite et des mélanges correspondants.

13. Matériau de remplacement d'os selon la revendication 11 ou 12, **caractérisé en ce que** le solide présent une proportion de 20 % en poids à 80 % en poids, de préférence 30 % en poids à 70 % en poids, par rapport au poids total de la masse.

14. Matériau de remplacement d'os selon l'une quelconque des revendications précédentes, **caractérisé en ce que** les corps de soutien et la masse présentent un matériau résorbable identique, le matériau résorbable étant de préférence un composé du calcium et/ou un composé du magnésium, en particulier un composé de type phosphate de calcium et/ou un composé de type phosphate de magnésium.

15. Matériau de remplacement d'os selon l'une quelconque des revendications précédentes, **caractérisé en ce que** la masse présente en outre un liquide porteur organique non soluble dans l'eau, en particulier choisi dans le groupe comprenant le triacétate de glycérine, le tributyrate de glycérine, le trioléate de glycérine, le dioléate de glycérine, le monooléate de glycérine, le caprate de caprylyle, l'oléate de décyle, le myristate d'isopropyle, le palmitate d'isopropyle, l'acide oléique, l'alcool oléylique, l'oléate d'oléyle, des triglycérides à chaîne courte, des triglycérides à chaîne moyenne, des esters d'acides gras à chaîne courte et moyenne du propylèneglycol, le benzoylacétate d'éthyle, le butyrate d'éthyle, le butyrylacétate d'éthyle, l'oléate d'éthyle, le caproate d'éthyle, le caprylate d'éthyle, le caprate d'éthyle, le laurate d'éthyle, le lévulinate d'éthyle, le myristate d'éthyle, le palmitate d'éthyle, le linoléate d'éthyle, le stéarate d'éthyle, l'acide ricinoléique, l'acide linoléique, l'acide linoléique, l'acide arachidique, l'acide oléique, l'arachidate d'éthyle, le α-tocophérol, le β-tocophérol, le γ-tocophérol, le δ-tocophérol, l'alcool benzylique, le benzoate de benzyle, le malonate de diéthyle butyle, le dibutyléther de diéthylèneglycol, le malonate de diéthyle, le malonate de diéthyle phényle, le phtalate de diéthyle, le sébaçate de diéthyle, le subarate de diéthyle, le succinate de diéthyle, le maléinate de dibutyle, le phtalate de dibutyle, une lécithine, une huile de paraffine, un pétrolatum, des paraffines liquides, des esters de l'acide sébacique, en particulier des esters de dibutyle de l'acide sébacique, des esters de diéthyle de l'acide sébacique, des esters de diisopropyle de l'acide sébacique, des esters de dioctyle de l'acide sébacique et des mélanges correspondants.
